(19) Europäisches Patentamt
European Patent Office
Office européen des brevets

(11) Veröffentlichungsnummer: **0 292 643 B1**

(12) **EUROPÄISCHE PATENTSCHRIFT**

(45) Veröffentlichungstag der Patentschrift: **16.09.92**

(51) Int. Cl.5: **C07C 405/00**, A61K 31/557

(21) Anmeldenummer: **88102189.3**

(22) Anmeldetag: **15.02.88**

# (54) Prostaglandin E1-Derivate und ihre therapeutische Anwendung.

(30) Priorität: **16.02.87 DE 3704825**

(43) Veröffentlichungstag der Anmeldung:
**30.11.88 Patentblatt 88/48**

(45) Bekanntmachung des Hinweises auf die Patenterteilung:
**16.09.92 Patentblatt 92/38**

(84) Benannte Vertragsstaaten:
**AT BE CH DE ES FR GB GR IT LI LU NL SE**

(56) Entgegenhaltungen:
**EP-A- 0 132 027**
**EP-A- 0 225 044**
**US-A- 3 735 005**

**CHEMICAL ABSTRACTS, Band 95, Nr. 25, 21. Dezember 1981, Seite 74, Zusammenfassung Nr. 215404g; Columbus, Ohio, US B. Schoelkens et al.: "Cardiovascular and antiplatelet activities of 6-keto-PGE1and 6-keto-PGA1 analogs" & PROSTAGLANDINS THROMBOXANES, PROC. INT. SYMP. PROSTAGLANDINS THROMBOXANES CARDIOVASC. SYST., 3rd, 1980 (pub. 1981)**

**CHEMICAL ABSTRACTS, Band 72, Nr. 13, 30. März 1970, Seite 306, Zusammenfassung Nr. 70628w; Columbus, Ohio, US & ZA-A-69 00 272 ( MAY AND BAKER LTD. ) 26-06-1969**

(73) Patentinhaber: **Frölich, Jürgen C., Prof. Dr. med.**
**Röhrichtweg 11**
**W-3000 Hannover 71(DE)**

Patentinhaber: **Bippi, Herbert, Dipl.-Biol.**
**Warburghof 10 B**
**W-3000 Hannover 61(DE)**

(72) Erfinder: **Frölich, Jürgen C., Prof. Dr. med.**
**Röhrichtweg 11**
**W-3000 Hannover 71(DE)**
Erfinder: **Bippi, Herbert, Dipl.-Biol.**
**Warburghof 10 B**
**W-3000 Hannover 61(DE)**

(74) Vertreter: **Vossius & Partner**
**Siebertstrasse 4 P.O. Box 86 07 67**
**W-8000 München 86(DE)**

EP 0 292 643 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Prostaglandin E1-Derivate (PGE1-Derivate) enthaltende Arzneimittel zur transkutanen Applikation.

Aus der DE-A-27 53 986 und der entsprechenden US-A-4,205,178 sind 6-Keto-Prostaglandin-E1-Derivate, insbesondere der 6-Keto PGE1-Methylester bekannt. Für diese Verbindungen wird eine Reihe von biologischen und pharmakologischen Wirkungen beschrieben. In Anhängigkeit von der zu behandelnden Erkrankung werden verschiedene Applikationsarten genannt, wie orale, intravenöse, subkutane, intraarterielle, buccale, rektale oder intravaginale Gabe. Topische Anwendung wird im Zusammenhang mit Hautbeschädigungen oder Erkrankungen an oder nahe der Stelle der Verletzung oder Erkrankung beschrieben.

6-Keto-Prostaglandin E1-Derivate sind ferner in der DE-A-28 40 032 beschrieben, in der ebenfalls auf verschiedene pharmakologische Wirkungen und Darreichungsformen hingewiesen wird.

Prostaglandin E1 (PGE1) sowie 6-Ketoprostaglandin E1 (6-k-PGE1) kommen zur Behandlung einer Reihe von Krankheiten in Frage. Dies sind u.a. periphere Verschlußkrankheit, Komplikationen bei Arteriosklerose wie Morbus Meniere und Hörsturz, akuter Myokardinfarkt, instabile Angina pectoris, akuter ischämischer Schlaganfall, Impotentia coeundi, Asthma bronchiale, Haarwuchsstörungen, sowie Abstoßungsreaktionen nach Nierentransplantationen; vgl. H. Sinzinger und W. Rogatti, Prostaglandin E1 in Atherosclerosis Springer Verlag Berlin Heidelberg - New York, 1986; A. Schrey, PGE1, Therapie der arteriellen Verschlußkrankheit, Universitätsdruckerei und Verlag Dr. C. Wolf und Sohn, München, 1985. PGE1 wird in der Medizin für die Behandlung chronischer arterieller Verschlußkrankheit im Stadium III und IV eingesetzt. Für diese Indikation ist eine intraarterielle bzw. intravenöse Infusion notwendig. Dies bedeutet eine erhebliche Einschränkung für die Anwendbarkeit von PGE1, da die Infusion für den Patienten belastend und mit einem gewissen Risiko arterieller Blutungen verknüpft ist. Beide Arten der Applikation (i.a. und i.v.) schließen eine ambulante Dauertherapie aus, die jedoch aufgrund der Erkrankungen angezeigt ist. Eine orale Anwendung von PGE1 ist regelmäßig problematisch, weil entweder die sehr niedrige Bioverfügbarkeit eine derartige Applikation verbietet, oder weil die typischen unerwünschten Wirkungen (Übelkeit, Erbrechen, Durchfall) wegen der hoben Konzentration bei oraler Verabreichung im Magen-Darm-Trakt prohibitiv sind.

EP-A-132 027 offenbart Prostaglandin $E_1$-Alkylester enthaltende Fettemulsionen, die eine blutdrucksenkende Wirkung zeigen. Insbesondere werden Methyl-, Ethyl-, n-Propyl-, Isopropyl-, n-Butyl, Isobutyl- und tert.-Butylester genannt.

C.A. 72: 70628w offenbart die Wirkung von $PGE_1$-Estern als Bronchodilatator. Insbesondere wird $PGE_1$-Methylester genannt.

Der Erfindung liegt die Aufgabe zugrunde, PGE1-Derivate als Arzneistoffe bzw. pharmakologische Wirkstoffe zur transkutanen Applikation zur Verfügung zu stellen. Die PGE1-Derivate, die als pharmakologische Wirkstoffe insbesondere zur transkutanen Applikation entwickelt wurden, werden durch die Haut resorbiert und nach Resorption durch Hydrolasen in Prostaglandin E1 bzw 6-Keto-PGE1 sowie Alkohol gespalten. Die PGE1-Derivate erfüllen damit das "Pro-Drug"-Konzept und vermeiden die Nachteile von PGE1 und 6-Keto-PGE1 bei arterieller, intravenöser oder oraler Applikation.

Gegenstand der Erfindung ist somit die Verwendung von Prostaglandin E1-Derivaten der allgemeinen Formel I

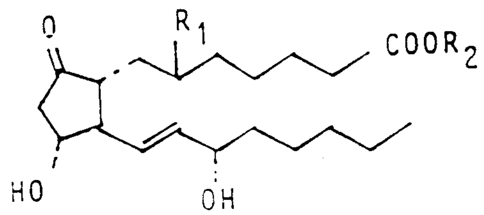

(I)

in der $R_1$ ein Wasserstoffatom (PGE1) oder ein Carbonylsauerstoffatom (6-k-PGE1) bedeutet und $R_2$ einen $C_{1-4}$-Alkylrest darstellt, zur Herstellung eines Arzneimittels zur transkutanen Applikation.

Beispiele für Alkylreste sind die Methyl-, Äthyl-, n-Propyl-,. Isopropyl-, n-Butyl-, Isobutyl- und tert.-Butylgruppe.

Der bevorzugte Rest $R_2$ ist wegen der Ungiftigkeit der Spaltprodukte die Äthylgruppe.

Die Herstellung der Verbindungen der allgemeinen Formel I erfolgt nach an sich bekannten Methoden durch Veresterung von PGE1 bzw. 6-k-PGE1. Beispielsweise wird der Methyl- und Äthylester durch Umsetzung mit Diazomethan bzw. Diazoäthan hergestellt; vgl. auch Ch. J. Sih et al., J.Am.Chem.Soc., Bd.

97 (1975), 857-865.

Die Verbindungen der allgemeinen Formel I können zur Behandlung von Durchblutungsstörungen, z.B. des Hirns, Herzens und der Extremitäten, zur Hemmung der Plättchenaggregation (Thrombozyten-Aggregation),zur Behandlung der Impotentia coeundi und zur Behandlung von allergischen Reaktionen wie Asthma bronchiale und Abstoßungsreaktionen bei Transplantationen, sowie bei Haarwuchsstörungen eingesetzt werden. Typische Beispiele für Störungen der Hirndurchblutung sind transitorische zerebrale Ischämie, Hörsturz, Schwindelzustände auf der Basis von Durchblutungsstörungen und ischämischer Schlaganfall. Typische Beispiele für Störungen der Herzdurchblutung sind Angina pectoris und Herzinfarkt. Typische Beispiele für Störungen der Durchblutung der Extremitäten sind periphere arterielle Durchblutungsstörungen bei Arteriosklerose, M. Raynaud und Raynaud-Syndrom.

Ferner können die Verbindungen der allgemeinen Formel I zur Behandlung gastrointestinaler Ulcera und Hautulcerationen benutzt werden. Typische Beispiele für gastrointestinale Ulcera sind Ulcus ventriculi, Ulcus duodeni und Colitis ulcerosa (Morbus Crohn). Ein typisches Beispiel für Hautulcerationen ist ulcus cruris. Die Verbindungen der allgemeinen Formel I haben eine zytoprotektive Wirkung. Zellen werden gegenüber Noxen widerstandsfähiger.

Die Verbindungen der allgemeinen Formel I können auch zur Behandlung von Hämatomen, insbesondere oberflächlich liegenden Hämatomen eingesetzt werden.

Die Herstellung von Arzneimitteln erfolgt nach üblichen Methoden. Zur Herstellung von Arzneimitteln zur transkutanen Applikation können die Verbindungen der allgemeinen Formel I in eine Gelgrundlage, Salbengrundlage bzw. flüssige Grundlage ohne oder mit verschiedenen Lösungsvermittlern sowie Stabilisatoren eingebracht werden. Als Verpackung werden Sprays, Tuben, Ampullen sowie Einzelportionen verwendet. Nach dem Auftragen auf die Haut oder mit zusätzlichem Okklusionsverband wird der Wirkstoff resorbiert.

Die Verbindungen der allgemeinen Formel I können auch mit oder ohne Stabilisatoren sowie Lösungsvermittler auf ein Pflaster aufgebracht und als solches appliziert werden.

Die Umwandlung des Äthylesters zu PGE1 im menschlichen Organismus wurde wie folgt nachgewiesen. Isotopen-markierter PGE1-Äthylester wurde wie vorstehend beschrieben aufgetragen. Die isotopenmarkierten Urinmetabolite wurden mit Hilfe von HPLC aufgetrennt und mit der Retentionszeit des Hauptmetaboliten von PGE1 7$\alpha$-Hydroxy-5,11-diketo-tetranorprosta-1,20-dionsäure verglichen. Es zeigte sich, daß der Hauptmetabolit nach Gabe des PGE1-Äthylesters mit dem Hauptmetaboliten nach Gabe von PGE1 identisch ist. Damit ist erwiesen, daß der PGE1-Äthylester Prodrug von PGE1 ist.

Die Beispiele erläutern die Erfindung.

**Beispiel 1**

Herstellung von Prostaglandin E1-äthylester

500$\mu$g PGE1 (1,31 $\mu$Mol) in 500 $\mu$l Äthanol werden mit einem Überschuß Diazoäthan in Diäthyläther (17 mg/ml; 0,3 mMol) in der Kälte unter Rühren versetzt. Danach läßt man das Reaktionsgemisch unter Rühren auf Raumtemperatur erwärmen und rührt anschließend noch 30 Minuten. Überschüssiges Diazoäthan sowie das Äthanol werden unter Stickstoff als Schutzgas bei Raumtemperatur entfernt. Der erhaltene Ester wird mittels Hochdruckflüssigkeitschromatographie (z.B. RP 18) gereinigt.

In analoger Weise werden 500 $\mu$g 6-Keto-PGE1 in 500 $\mu$l Äthanol mit einem Überschuß Diazoäthan in Diäthyläther umgesetzt und aufgearbeitet. Der Ester wird durch Hochdruckflüssigkeitschromatographie (RP 18) gereinigt.

**Beispiel 2**

250 $\mu$g Prostaglandin E1-äthylester in 250 $\mu$l Äthanol wurden zusammen mit isotopen-markiertem PGE1 Äthylester in 2 g einer Gelgrundlage der nachstehend angegebenen Zusammensetzung eingearbeitet. Das Gel wurde am Oberarm aufgetragen und 1 Minute einmassiert. Die Auftragsfläche wurde mit einer Kunststoff-Folie überlappend abgedeckt.

Zu einem 1 Woche später liegenden Zeitpunkt wurden 250 $\mu$g Prostaglandin E1 in 250 $\mu$l Äthanol zusammen mit isotopen-markiertem PGE1 in 2 g Gelgrundlage der nachstehend angegebenen Zusammensetzung eingebracht und ebenfalls am Oberarm aufgetragen und 1 Minute einmassiert. Die Messung der resorbierten Menge erfolgte durch Bestimmung der isotopen-markierten Prostaglandin-Metabolite im Urin. Hierzu wurde ab Applikationsbeginn der Urin quantitativ portioniert gesammelt. Die Kunststoff-Folie wurde nach 4 Stunden entfernt und das restliche Gel abgewischt. Wie aus Fig. 1 ersichtlich ist, war die Resorption von PGE1-Äthylester (23%) gegenuber PGE1 (ca. 4%) deutlich verbessert.

Die Gelgrundlage wurde nach folgender Rezeptur hergestellt:

| | |
|---|---|
| Isopropanol | 40,0 g |
| Diisopropyladipat | 0,5 g |
| Carbopol 940 | 2,0 g |
| Trometamol | 1,91 g |
| Gereinigtes Wasser | ad 100,0 g |

Isopropanol kann auch durch Äthanol ersetzt werden.

Wasser, Alkohol und Diisopropyladipat werden miteinander gemischt, das Carbopol wird in dieser Mischung dispergiert und quellen gelassen. Das Gel wird mit der wäßrigen Trometamol-Lösung neutralisiert.

**Patentansprüche**

**Patentansprüche für folgende Vertragsstaaten : AT, BE, CH, DE, FR, GB, IT, LI, LU, NL, SE**

**1.** Verwendung von Prostaglandin E1-Derivaten der allgemeinen Formel I

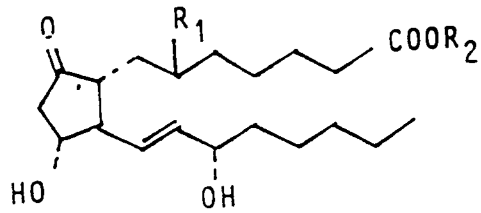

(I)

in der $R_1$ ein Wasserstoffatom oder ein Carbonylsauerstoffatom bedeutet und $R_2$ einen $C_{1-4}$-Alkylrest darstellt zur Herstellung eines Arzneimittels zur transkutanen Applikation.

**2.** Ausführungsform nach Anspruch 1, wobei $R_2$ die Äthylgruppe bedeutet.

**3.** Ausführungsform nach Anspruch 1 oder 2, wobei das Arzneimittel zur transkutanen Applikation zur Behandlung von Durchblutungsstörungen Impotentia coeundi, zur Hemmung der Plättchenaggregation, zur Behandlung von Asthma bronchiale gastrointestinaler Ulcera und Hautulcerationen, von Hämatomen, Haarwuchsstörungen oder von Abstoßungsreaktionen und Transplantation eines Organs bestimmt ist.

**Patentansprüche für folgende Vertragsstaaten : ES, GR**

**1.** Verfahren zur Herstellung eines ein Prostaglandin E1-Derivat der allgemeinen Formel I

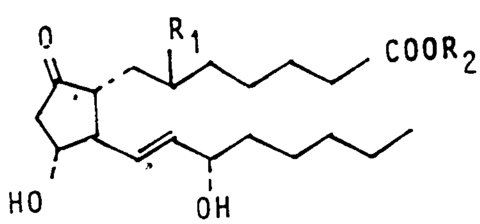

(I) ,

in der $R_1$ ein Wasserstoffatom oder ein Carbonylsauerstoffatom bedeutet und $R_2$ einen $C_{1-4}$-Alkylrest darstellt, enthaltenden Arzneimittels zur transkutanen Applikation, dadurch gekennzeichnet, daß man ein Prostaglandin E1-Derivat der allgemeinen Formel I mit einem zur transkutanen Applikation geeigneten Träger- oder Hilfsstoff verbindet.

**2.** Verfahren nach Anspruch 1, wobei $R_2$ die Äthylgruppe bedeutet.

**3.** Verfahren nach Anspruch 1 oder 2, wobei das Arzneimittel zur transkutanen Applikation zur Behandlung

von Durchblutungsstörungen, Impotentia coeundi, zur Hemmung von Plättchenaggregation, zur Behandlung von Asthma bronchiale, von gastrointestinaler Ulcera und Hautulcerationen, von Hämatomen, Haarwuchsstörungen oder von Abstoßungsreaktionen und Transplantation eines Organs bestimmt ist.

## Claims

**Claims for the following Contracting States : AT, BE, CH, DE, FR, GB, IT, LI, LU, NL, SE**

**1.** Use of prostaglandin E1 derivatives of general formula I

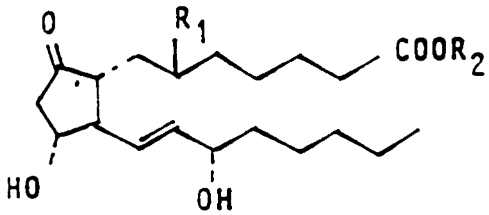

(I)

in which $R_1$ is a hydrogen atom or a carbonyl oxygen atom and $R_2$ is a $C_{1-4}$ alkyl residue for the preparation of a pharmaceutical composition to be administered transcutaneously.

**2.** An embodiment according to claim 1, wherein $R_2$ is the ethyl group.

**3.** An embodiment according to claim 1 or 2, wherein the pharmaceutical composition for transcutaneous administration is determined for the treatment of circulatory insufficiencies, of impotence, for the inhibition of platelet aggregation, for the treatment of bronchial asthma, of gastrointestinal ulcers and ulcers of the skin, of haemotomas, of impaired hair growth or of rejections following transplantations of an organ.

**Claims for the following Contracting States : ES, GR**

**1.** Process for the preparation of a pharmaceutical composition for transcutaneous administration containing a prostaglandin E1 derivative of general formula I

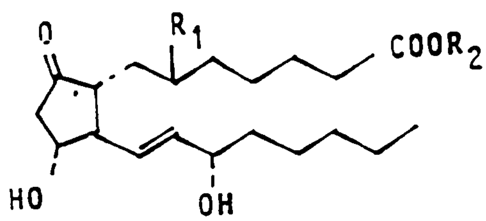

(I)

in which $R_1$ is a hydrogen atom or a carbonyl oxygen atom and $R_2$ is a $C_{1-4}$ alkyl residue, characterized in that a prostaglandin E1 derivative of general formula I is combined with a carrier substance or adjuvant suitable for transcutaneous administration.

**2.** A process according to claim 1, wherein $R_2$ is the ethyl group.

**3.** A process according to claim 1 or 2, wherein the pharmaceutical composition for transcutaneous administration is determined for the treatment of circulatory insufficiencies, of impotence, for the inhibition of platelet aggregation, for the treatment of bronchial asthma, of gastrointestinal ulcers and ulcers of the skin, of haemotomas, of impaired hair growth or of rejections following transplantations of an organ.

## Revendications

**Revendications pour les Etats contractants suivants : AT, BE, CH, DE, FR, GB, IT, LI, LU, NL, SE**

**1.** Utilisation de dérivés de prostaglandine E1 répondant à la formule générale I

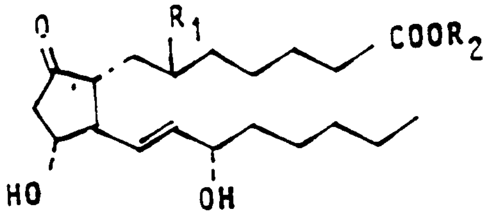

(I)

dans laquelle $R_1$ représente un atome d'hydrogène ou un atome d'oxygène carbonylique et $R_2$ représente un radical alkyle en $C_{1-4}$ pour la préparation d'un médicament destiné à une application transcutanée.

**2.** Forme de réalisation suivant la revendication 1, dans laquelle $R_2$ représente le groupe éthyle.

**3.** Forme de réalisation suivant l'une des revendications 1 et 2, dans laquelle le médicament est destiné à une application transcutanée pour le traitement d'une perturbation dans l'irrigation sanguine et de l'impuissance sexuelle, pour l'inhibition de l'agrégation des plaquettes, pour le traitement de l'asthme des bronches, des ulcères gastro-intestinaux, des ulcérations de la peau, des hématomes, des perturbations dans la croissance des cheveux ou des réactions de rejet après transplantation d'un organe.

**Revendications pour les Etats contractants suivants : ES, GR**

**1.** Procédé de préparation d'un médicament contenant un dérivé de prostaglandine E1 répondant à la formule générale I

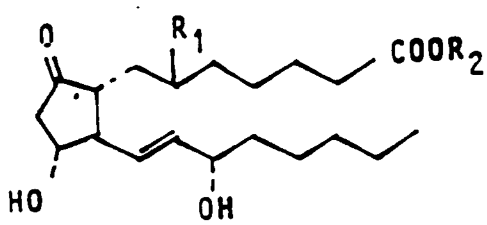

(I) ,

dans laquelle $R_1$ représente un atome d'hydrogène ou un atome d'oxygène carbonylique et $R_2$ représente un radical alkyle en $C_{1-4}$, pour une application transcutanée, caractérisé en ce qu'on combine un dérivé de prostaglandine E1 de la formule générale I avec un support ou adjuvant approprié pour une application transcutanée.

**2.** Procédé suivant la revendication 1, dans lequel $R_2$ représente le groupe éthyle.

**3.** Procédé suivant l'une des revendications 1 et 2, dans lequel le médicament prévu pour l'application transcutanée est destiné au traitement de troubles dans l'irrigation sanguine ou de l'impuissance sexuelle, pour l'inhibition de l'agrégation des plaquettes, pour le traitement de l'asthme des bronches, des ulcères gastrointestinaux, des ulcérations de la peau, des hématomes, des perturbations dans la croissance des cheveux ou des réactions de rejet après transplantation d'un organe.

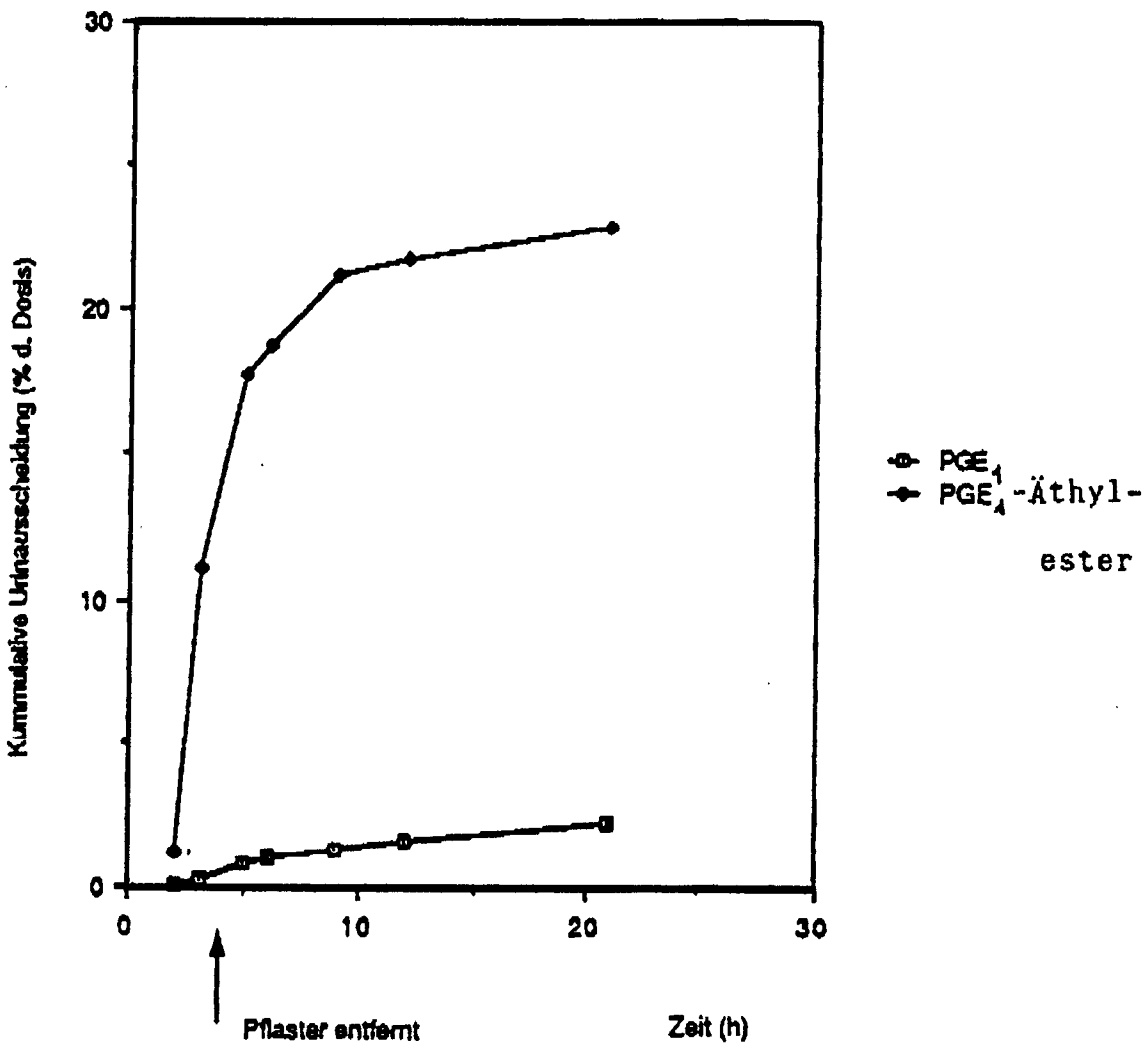
30
20
10
0
0
10
20
30
Kummulative Urinausscheidung (% d. Dosis)
Pflaster entfernt
Zeit (h)
PGE1
PGE1-Äthyl-
ester

Fig. 1